# EUROPEAN PATENT APPLICATION

(11) **EP 3 950 700 A1**
(43) Date of publication of application: **09.02.2022**
(21) Application number: 20776861.5
(22) Date of filing: 14.02.2020
(51) Int. Cl.: C07K 1/113, C07K 7/08, C07K 14/00, C40B 40/10, C40B 30/10

(54) **PEPTIDE COMPLEX AND PRODUCTION METHOD THEREFOR, AND USE OF SAID PEPTIDE COMPLEX**

(30) Priority: 28.03.2019 JP 2019064539
(71) Applicant: Kaneka Corporation, Osaka-shi, Osaka 530-8288 (JP)
(72) Inventor: TAKATSU, Keishi, Takasago-shi, Hyogo 676-8688 (JP); SHIKAKURA, Toshihiro, Takasago-shi, Hyogo 676-8688 (JP); MATSUDA, Yuka, Takasago-shi, Hyogo 676-8688 (JP); MOUTAI, Tatsuya, Takasago-shi, Hyogo 676-8688 (JP); MAEDA, Hirofumi, Takasago-shi, Hyogo 676-8688 (JP); KITA, Hiroshi, Takasago-shi, Hyogo 676-8688 (JP); KITANO, Mitsuaki, Takasago-shi, Hyogo 676-8688 (JP)
(74) Representative: Hoffmann Eitle
(86) International application number: PCT/JP2020/005762
(87) International publication number: WO 2020/195303

(57) **Abstract**

A peptide complex including: a peptide; and a cell-membrane-permeability-imparting group introduced into the peptide, wherein the cell-membrane-permeability-imparting group includes a dendritic structure including four or more branches, and at least four branches of the four or more branches include basic functional groups at ends thereof.

## Description

### Technical Field

The present invention relates to a peptide complex that is cell-membrane permeable, a method for producing the peptide complex, a peptide library including the peptide complex, and a method for screening for a functional peptide using the peptide library.

### Background Art

In recent years, peptide (polypeptide) libraries of various amino acid sequences have been used to select, for example, therapeutic drugs and molecules having high affinity to the target molecules.

Regarding methods for producing the peptide libraries, for example, the ribosome display method (RD method) using a ribosome display complex including a peptide chain, a mRNA molecule, and a ribosome is known as a method of an *in vitro* translation system (see, for example, Patent Literature 1). The RD method is very excellent and useful because it just mixes only an *in vitro* translation system and mRNA to be able to produce 10¹² or more kinds of peptide libraries in several minutes.

In drug development, techniques for efficient intracellular delivery of various active molecules such as proteins and nucleic acids are also important.

Known techniques for intracellular delivery of active molecules include, for example, a method of fusing cell-membrane-penetrating peptides containing many basic amino acids and a method of using a dendrimer which is a dendritic polymer having a structure that is regularly branched from the center (see, for example, Patent Literature 2).

When active molecules do not enter cells sufficiently in a short period of time, many of the active molecules are metabolized before entering cells. This may raise a risk that the active molecules will have difficulty in exhibiting pharmaceutical efficacy. Thus, it is preferable that the active molecules enter cells in a shorter time.

Nonetheless, any satisfactory technique for allowing peptides to enter cells in a short period of time has not been developed yet, and there is currently a strong demand for rapid development thereof.

### Citation List

### Patent Literature

PTL 1: Japanese Patent Application Laid-Open No. 2008-271903
PTL 2: US Patent No. 7,862,807

### Summary of Invention

### Technical Problem

The present invention aims to solve the above problems in the art and achieve the following object. Specifically, the present invention aims to provide a peptide complex that is excellent in entering cells in a short period of time, a method for producing the peptide complex, a peptide library including the peptide complex, and a method for screening for a functional peptide using the peptide library.

### Solution to Problem

The present inventors conducted intensive studies to achieve the above object and have found that a peptide complex can enter cells sufficiently in a short period of time by introducing to a peptide a cell-membrane-permeability-imparting group including a dendritic structure including four or more branches where at least four branches of the four or more branches include basic functional groups at ends thereof.

The present invention is based on the above finding obtained by the present inventors. Means for achieving the object are as follows.
<1> A peptide complex including:
   a peptide; and
   a cell-membrane-permeability-imparting group introduced into the peptide,
   wherein the cell-membrane-permeability-imparting group includes a dendritic structure including four or more branches, and
   at least four branches of the four or more branches include basic functional groups at ends thereof.
<2> A method for producing a peptide complex, the method including
   introducing a cell-membrane permeable molecule into a peptide,
   wherein the cell-membrane permeable molecule includes a dendritic structure including four or more branches, and at least four branches of the four or more branches include basic functional groups at ends thereof.
<3> A peptide library including
   the peptide complex defined in <1> above.
<4> A method for screening for a functional peptide, the method including
   screening for the functional peptide using the peptide library defined in <3> above.

### Advantageous Effects of Invention

According to the present invention, it is possible to solve the above problems in the art and achieve the above object, and to provide a peptide complex that is excellent in entering cells in a short period of time, a method for producing the peptide complex, a peptide library including the peptide complex, and a method for screening for a functional peptide using the peptide library.

### Description of Embodiments

### (Peptide complex)

The peptide complex of the present invention includes a peptide and a cell-membrane-permeability-imparting group introduced into the peptide. If necessary, the peptide complex further includes other components.

### <Peptide>

The peptide is not particularly limited and may be appropriately selected depending on the intended purpose as long as the cell-membrane-permeability-imparting group can be introduced into the peptide.

The kind of the amino acid in the peptide is not particularly limited and may be appropriately selected depending on the intended purpose. The amino acid may be a native amino acid or a non-native amino acid, or may be a D-amino acid or an L-amino acid.

The peptide may be a modified peptide such as a lipopeptide.

An amino acid residue used for introduction of the cell-membrane-permeability-imparting group (hereinafter this amino acid residue may be referred to as a "reactive amino acid residue") is not particularly limited and may be appropriately selected depending on the intended purpose. Examples of the amino acid residue include a cysteine residue, a lysine residue, a histidine residue, a tryptophan residue, a tyrosine residue, a serine residue, and a threonine residue. Also, an alcohol side chain in the amino acid residue may be used. These may be used alone or in combination.

The number of the reactive amino acid residues in the peptide is not particularly limited and may be appropriately selected depending on the intended purpose.

When, for example, a cyclic peptide is used as the peptide, the number of the reactive amino acid residues in the peptide is preferably two or more. The upper limit of the number of the reactive amino acid residues in the peptide is not particularly limited and may be appropriately selected depending on the intended purpose. The upper limit thereof is preferably 10 or less. This is because when there are many reaction points, the number and positions of the cell-membrane-permeability-imparting groups to be bound to the peptide are not stable, which may have difficulty in comparing the properties of the peptides derived from the amino acid sequences.

When, for example, cysteine residues in the peptide form a disulfide bond to contribute to stabilization of a higher order conformation of the peptide, it is preferable that the reactive amino acid residues be additionally introduced into the peptide.

The positions of the reactive amino acid residues in the peptide are not particularly limited and may be appropriately selected depending on the intended purpose.

When the peptide used is, for example, a ribosome display complex (hereinafter may be referred to as a "RD complex") including a mRNA molecule, a peptide chain translated therefrom (hereinafter may be referred to as a "polypeptide chain"), and a ribosome, the reactive amino acid residues are preferably in a region outside the exit tunnel of the ribosome, specifically between the 2^{nd} position from the N-terminus and the 30^{th} position from the C-terminus (the 2^{nd} position from the N-terminus and the 30^{th} position from the C-terminus are included). This is because modification reaction by the linker molecule cannot easily be inhibited sterically by the ribosome.

The position from the C-terminus is preferably the 50^{th} position from the C-terminus and more preferably 100^{th} position from the C-terminus.

When the position of the reactive amino acid residue is counted from the N-terminus, the position is, for example, between the 2^{nd} position and the 1,000^{th} position from the N-terminus, preferably between the 2^{nd} position and the 100^{th} position from the N-terminus, and more preferably between the 2^{nd} position and the 50^{th} position from the N-terminus, although the position can be appropriately set depending on the chain length of the peptide.

A method for producing the RD complex is not particularly limited and may be appropriately selected from known methods. Examples of the method include the method described in International Publication No. WO2017/213158. The RD complex can also be produced using a commercially available kit.

The amino acid sequence of the peptide is not particularly limited and may be appropriately selected depending on the intended purpose. In order for it to be useful as a peptide library, the amino acid sequence thereof preferably includes a random sequence at a specific position. From such a random sequence, a useful amino acid sequence can be identified depending on the predetermined purpose.

The position of the random sequence in the peptide is not particularly limited and may be appropriately selected depending on the intended purpose. When, for example, the RD complex is used, similar to the position of the reactive amino acid residue, the position of the random sequence is preferably between the 2^{nd} position from the N-terminus and the 30^{th} position from the C-terminus (the 2^{nd} position from the N-terminus and the 30^{th} position from the C-terminus are included). In other words, the reactive amino acid residue is preferably included in the random sequence. Thus, a preferable position of the random sequence can be set from the same range as the range of the preferable position of the reactive amino acid residue.

The number of the random sequences in the peptide may be one or be two or more. The upper limit of the number of the random sequences is not particularly limited and may be appropriately selected depending on the purpose. The upper limit thereof is preferably 10 or less.

The number of amino acid residues in one random sequence is not particularly limited and may be appropriately selected depending on the purpose. It may be, for example, 1 or more but 30 or less.

Diversity of the peptide library can be increased as the one random sequence becomes longer or the number of the random sequences becomes greater.

The peptide may further include, for example, a sequence for purification of the polypeptide chain, such as a FLAG (registered trademark) sequence or a poly His sequence, a sequence that is selectively cut by, for example, a protease, and a spacer sequence.

The number of the amino acid residues of the peptide is not particularly limited and may be appropriately selected depending on the intended purpose. It can be set to, for example, 10 or more but 5,000 or less. The upper limit thereof is preferably 800 or less, more preferably 400 or less, and particularly preferably 200 or less.

A method for synthesizing the peptide is not particularly limited and may be appropriately selected from known methods.

### <Cell-membrane-permeability-imparting group>

The cell-membrane-permeability-imparting group is, for example, represented by General Formula (1) below.

A-B-C ... General Formula (1)

In the General Formula (1), "A" denotes a group for linking to the peptide, "B" denotes a linking group or a single bond, and "C" denotes a group including a dendritic structure.

### -Group for linking to the peptide-

The group for linking to the peptide contributes to introduction of the cell-membrane-permeability-imparting group into the peptide through reaction with the reactive amino acid residue in the peptide.

The reactive amino acid residue is an amino acid residue that reacts with the group for linking to the peptide. The reactive amino acid residue may be an amino acid residue that directly reacts with the group for linking to the peptide, or may be an amino acid residue that is modified to be able to react with the group for linking to the peptide.

The group for linking to the peptide is not particularly limited and may be appropriately selected depending on the intended purpose as long as it can be linked to the peptide. Examples thereof include groups that can form bonds through reaction with a thiol group of a cysteine residue, a side-chain amino group (-NH₂) of a lysine residue, and a side-chain amino group (>NH) of a histidine residue or a tryptophan residue.

Specific examples of the group for linking to the peptide include a halogenated alkyl group, an activated carbonyl group, an unsaturated hydrocarbon group, an epoxy group, a sulfonyl-containing group, an isocyanate group, a thioisocyanate group, a carbene-generating group, a disulfide bond-containing group, and a thiol group described in paragraphs [0067] to [0076] of International Publication No. WO2017/213158.

When, for example, the oxime ligation method is used to bind the peptide and the cell-membrane-permeability-imparting group, a group expressed by the following structural formula can also be used as the group for linking to the peptide.

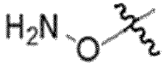

### -Linking group-

The linking group is a group that links the group for linking to the peptide and the group including the dendritic structure.

The structure of the linking group is not particularly limited and may be appropriately selected depending on the intended purpose.

### -Group including the dendritic structure-

The cell-membrane-permeability-imparting group includes a dendritic structure including four or more branches where at least four branches of the four or more branches includes basic functional groups at the ends thereof.

The group including the dendritic structure is a group that imparts cell-membrane permeability to the peptide.

The number of the branches in the dendritic structure of the cell-membrane-permeability-imparting group is not particularly limited and may be appropriately selected depending on the intended purpose as long as it is four or more. Examples of the number of the branches include six or more and nine or more.

The "including four or more branches" refers to the total number of the branches of the dendritic structure in the cell-membrane-permeability-imparting group. Specifically, the cell-membrane-permeability-imparting group may include one dendritic structural unit including four or more branches, or may include two or more dendritic structural units each including three or less branches so that the total number of the branches in the two or more dendritic structural units is four or more.

The number of the dendritic structural units in the cell-membrane-permeability-imparting group is not particularly limited and may be appropriately selected depending on the intended purpose. It may be one or be two or more.

The number of the branches in the one dendritic structural unit is not particularly limited and may be appropriately selected depending on the intended purpose. It is preferably three or more.

The structure of the branch of the dendritic structure is not particularly limited and may be appropriately selected depending on the intended purpose.

A method for producing the group including the dendritic structure is not particularly limited and may be appropriately selected depending on the intended purpose. The group including the dendritic structure can be produced by the methods described in, for example, U.S. Patent No. 7,862,807.

### --Basic functional group--

The basic functional group is not particularly limited and may be appropriately selected depending on the intended purpose. Examples thereof include a guanidino group, an amino group, and an imidazole group. Of these, a guanidino group is preferably included. These basic functional groups may be used alone or in combination.

The number of the basic functional groups is not particularly limited and may be appropriately selected depending on the intended purpose as long as it is four or more. Examples of the number of the basic functional groups include six or more and nine or more.

Preferably, four or more guanidino groups are included as the basic functional groups.

The positions of the basic functional groups in the cell-membrane-permeability-imparting group are not particularly limited and may be appropriately selected depending on the intended purpose as long as the basic functional groups are at least positioned at the ends of four branches of the dendritic structure.

Specific examples of the dendritic structural unit of the group including the dendritic structure include those represented by General Formula (C) below.

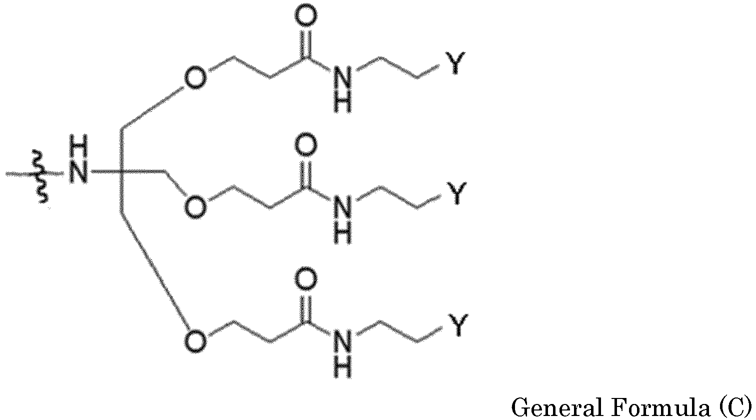

The above General Formula (C) is a dendritic structural unit including three branches. In the General Formula (C), "Y" denotes the basic functional group.

The basic functional group may be formed at the ends of all of the branches or may be formed at the ends of some of the branches.

The cell-membrane-permeability-imparting group is introduced into the peptide through reaction between the group for linking to the peptide in the cell-membrane-permeability-imparting group and the reactive amino acid residue in the peptide. During the reaction, the structure of the group for linking to the peptide changes.

Specific examples of the cell-membrane-permeability-imparting group include a compound of the following structural formula.

The cell-membrane-permeability-imparting group expressed by the following structural formula is an example including as the group for linking to the peptide, a group that can bind to the peptide by the oxime ligation method and as the group including the dendritic structure, two dendritic structural units each including guanidino groups as the basic functional groups at all of the ends of three branches, where the group for linking to the peptide is linked via the linking group to the group including the dendritic structure.

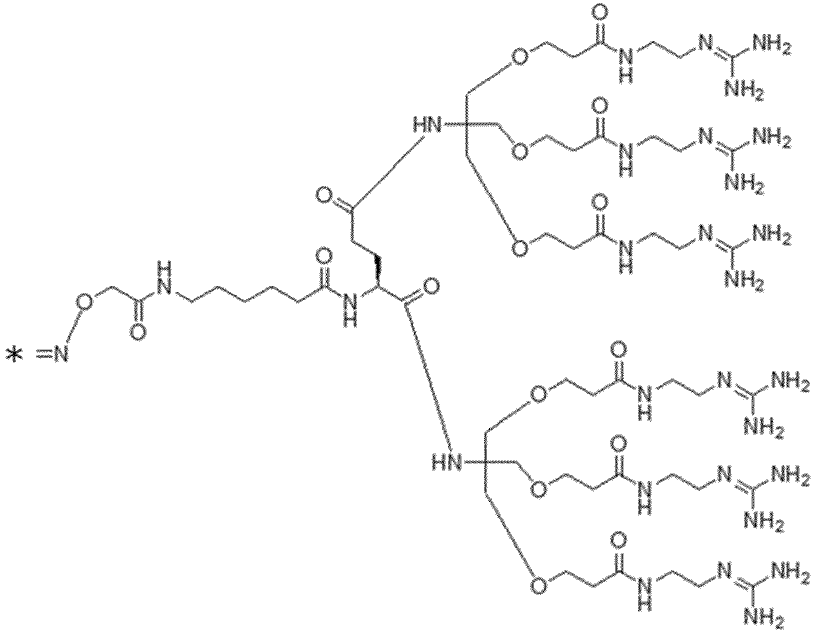

In the structural formula, "*" denotes a linking site to the peptide.

The cell-membrane-permeability-imparting group expressed by the following structural formula is an example including as the group for linking to the peptide, a group that can bind to the peptide by the oxime ligation method and as the group including the dendritic structure, three dendritic structural units each including guanidino groups as the basic functional groups at all of the ends of three branches, where the group for linking to the peptide is linked via the linking group to the group including the dendritic structure.

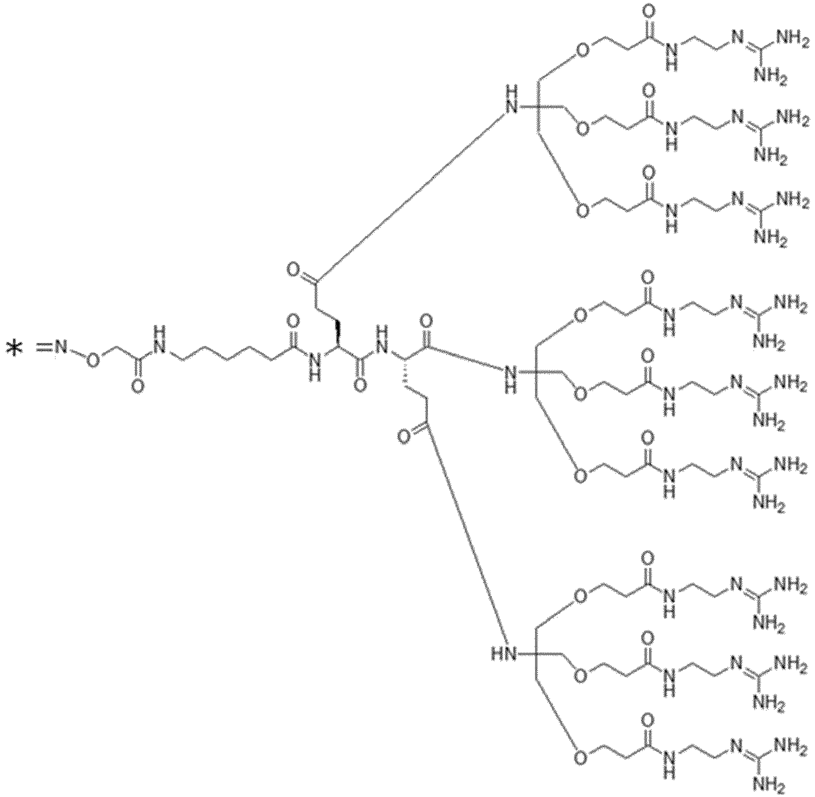

In the structural formula, "*" denotes a linking site to the peptide.

### <Other components>

The other components in the peptide complex are not particularly limited and may be appropriately selected depending on the intended purpose as long as they do not impair the effects of the present invention. Examples thereof include luminescent substances such as fluorescent substances, dyes, radioactive substances, drugs, toxins, nucleic acids, amino acids, saccharides, lipids, and various polymers. These may be used alone or in combination.

The fluorescent substance is not particularly limited and may be appropriately selected depending on the intended purpose. Examples thereof include fluorescent dyes such as fluoresceins, rhodamines, coumarins, pyrenes, and cyanines.

The other components can be bound to the above peptide, for example, directly or via a linking group.

The peptide complex of the present invention is excellent in entering cells in a short period of time. Therefore, for example, screening a peptide complex library including random sequences can identify a useful amino acid sequence that is, for example, excellent in entering cells in a short period of time and high in affinity to the target substance.

### (Method for producing a peptide complex)

A method of the present invention for producing a peptide complex includes an introduction step of introducing a cell-membrane permeable molecule into a peptide. If necessary, the method further includes other steps.

### <Introduction step>

The introduction step is a step of introducing a cell-membrane permeable molecule into a peptide (hereinafter the introducing may be referred to as "binding", "inserting", or "linking")

By the introduction step, the cell-membrane-permeability-imparting group in the section (Peptide complex) described above can be introduced into the peptide.

In the introduction step, it is enough that the cell-membrane-permeability-imparting group is introduced into at least one peptide in the reaction product. It is, however, preferable that the cell-membrane-permeability-imparting group be introduced into all of the peptides.

### -Peptide-

The peptide is similar to those described in the section <Peptide> of (Peptide complex) described above. The peptide may be a peptide library.

### -Cell-membrane permeable molecule-

The cell-membrane permeable molecule is introduced into the peptide and exists as the cell-membrane-permeability-imparting group in the section (Peptide complex) described above. The cell-membrane permeable molecule can be similar to those described in the section <Cell-membrane-permeability-imparting group> of (Peptide complex) described above.

A method for producing the cell-membrane permeable molecule is not particularly limited and may be appropriately selected depending on the intended purpose. Examples of the method include a method including a step of forming a precursor of the cell-membrane-permeability-imparting group, a step of introducing a linking group including a precursor of the group for linking to the peptide into the precursor of the cell-membrane-permeability-imparting group, a step of forming the group for linking to the peptide from the precursor of the group for linking to the peptide, and a step of forming the cell-membrane-permeability-imparting group from the precursor of the cell-membrane-permeability-imparting group.

Each of the above steps can be performed by a method that is appropriately selected from known techniques for chemical synthesis.

### -Introduction-

A method of the introduction is not particularly limited and may be appropriately selected depending on the intended purpose. Examples of the method include a method of reacting the group for linking to the peptide in the cell-membrane permeable molecule with the reactive amino acid residue in the peptide.

Conditions such as the temperature and time of the reaction are not particularly limited and may be appropriately selected depending on the intended purpose.

### <Other steps>

The other steps are not particularly limited and may be appropriately selected depending on the intended purpose as long as they do not impair the effects of the present invention. Examples thereof include a step of purifying the peptide complex.

A method for confirming whether the obtained peptide complex has a desired structure is not particularly limited and may be appropriately selected from known analytical methods. Examples of the method include analytical methods such as mass spectrometry, proton nuclear magnetic resonance spectroscopy, ¹³C nuclear magnetic resonance spectroscopy, UV spectroscopy, and infrared spectroscopy.

### (Peptide library)

A peptide library of the present invention includes the peptide complex of the present invention. If necessary, the peptide library further includes other components.

The peptide library may consist of the peptide complex of the present invention, or may include a peptide into which the cell-membrane-permeability-imparting group is not introduced.

The peptide library can be produced in the same manner as in the (Method for producing a peptide complex) described above.

### (Method for screening for functional peptide)

The method of the present invention for screening for a functional peptide includes a step of screening for a functional peptide using the peptide library of the present invention. If necessary, the method further includes other steps.

A method of the screening is not particularly limited and may be appropriately selected from known methods as long as the method uses the peptide library of the present invention. Examples of the method include a method of screening for the functional peptide having affinity to a desired target substance by repeating a step including mixing the target substance and the peptide library, selecting a bound peptide complex (e.g., a RD complex), dissociating RNA from the RD complex, preparing DNA from the RNA, amplifying the DNA, transcribing the DNA to mRNA, and again preparing a RD complex library.

### Examples

The present invention will be described below in more detail by way of, for example, Examples. The present invention, however, should not be construed as being limited to, for example, these Examples.

### (Comparative Example 1: Synthesis of peptide complex G3-P)

### <Synthesis of Compound C1>

Following the method described in The Journal of Organometallic Chemistry 2013, 17-24, Compound C1 expressed by the following structural formula was synthesized. In the structural formula of the compound, "Fmoc" denotes a "9-fluorenylmethyloxycarbonyl group".

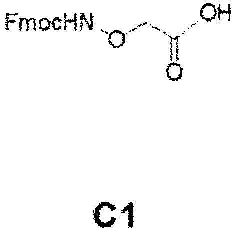

### <Synthesis of Compound C3>

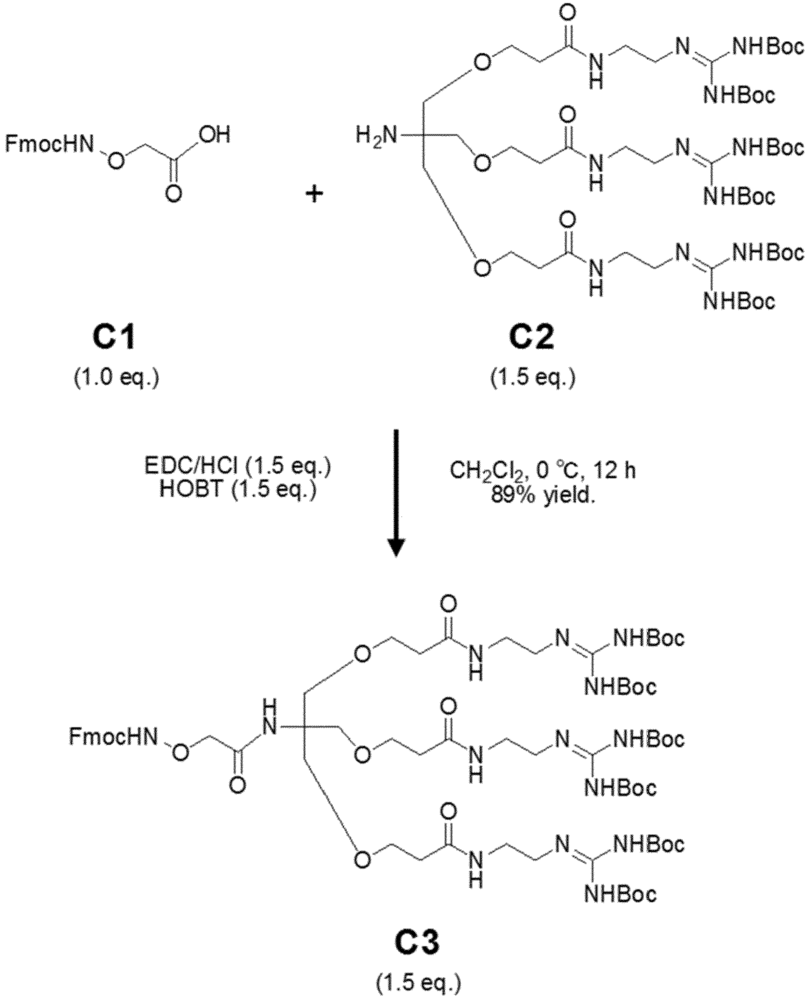

As presented in the above reaction scheme, Compound C3 expressed by the above structural formula was synthesized.

Specifically, Compound C1 (50 mg, 0.159 mmol) was dissolved in methylene chloride (5 mL) and the solution was cooled to 0°C. Compound C2 (284 mg, 0.239 mmol), which had been synthesized by the same method as the method described in U.S. Patent No. 7,862,807, 1-(3-dimethylaminopropyl)-3-ethylcarbodiimide hydrochloride (EDC/HCl) (45.8 mg, 0.239 mmol), and 1-hydroxybenzotriazole (HOBT) (32.2 mg, 0.239 mmol) were added, and the mixture was stirred at 0°C for 12 hours. A saturated sodium chloride solution (20 mL) was added, and the mixture was extracted with methylene chloride (three times with 20 mL per time). The organic phase was dried with magnesium sulfate, followed by filtration and then concentration. The residue was purified through preparative thin-layer chromatography (PTLC) (methylene chloride/methanol (MeOH)=10/1; Rf=0.7) to obtain Compound C3 (210 mg, 0.141 mmol, yield: 89%) as colorless oily matter.

In the structural formulae of the compounds, "Fmoc" denotes a "9-fluorenylmethyloxycarbonyl group" and "Boc" denotes a "tert-butoxycarbonyl group".

Identification data of the Compound C3 through 1H NMR were as follows. 1H NMR(CDCl₃): δ 8.58(t, ³*J*_{HH}=6.0Hz, 3H), 7.74(d, ³*J*_{HH}=8.0Hz, 2H), 7.58-7.57(m, 4H), 7.38(t, ³*J*_{HH}=7.5Hz, 2H), 7.28(t, ³*J*_{HH}=7.0Hz, 2H), 4.47(d, ³*J*_{HH}=7.0Hz, 2H), 4.26(s, 6H), 3.68(t, ³*J*_{HH}=5.5Hz, 6H), 3.49-3.46(m, 6H), 3.37-3.33(m, 6H), 2.43(t, ³*J*_{HH}=6.0Hz, 6H), 1.49(s, 27H), 1.46(s, 27H)

### <Synthesis of Compound C4>

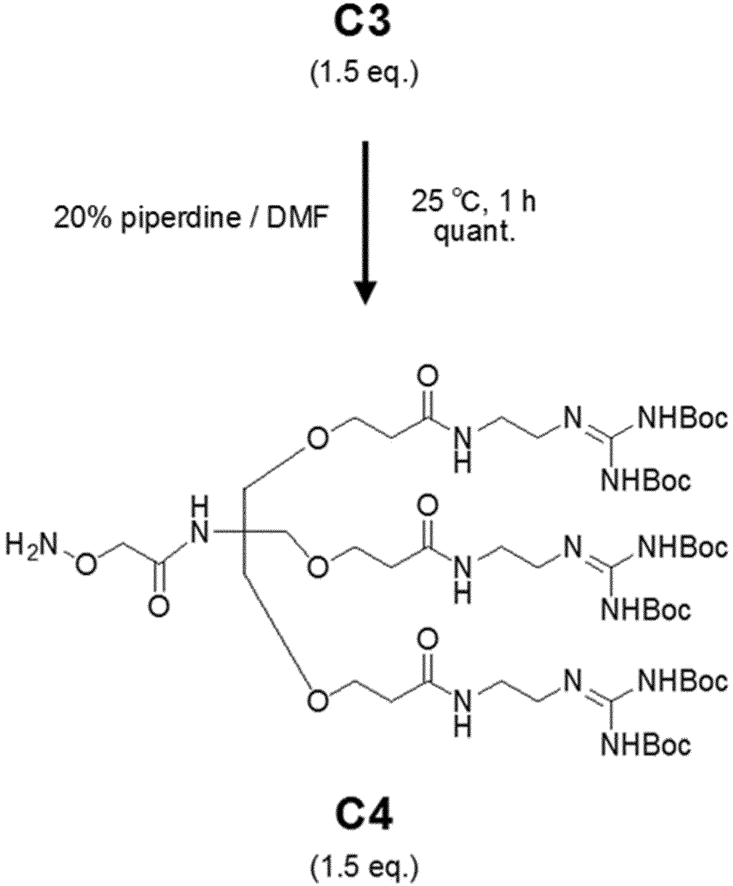

As presented in the above reaction scheme, Compound C4 expressed by the above structural formula was synthesized.

Specifically, Compound C3 (210 mg, 0.141 mmol) and a 20% piperidine/N,N-dimethylformamide (DMF) solution (1.0 mL) were mixed, and the mixture was stirred at 25°C for 1 hour. Nitrogen gas was blown to the reaction liquid to volatilize the solvent. The residue was purified through PTLC (methylene chloride/MeOH=10/1) to obtain Compound C4 (168 mg, 0.133 mmol, yield: 94%) as colorless transparent oily matter.

Identification data of the Compound C4 through 1H NMR were as follows. 1H NMR(CDCl₃): δ 11.4(s, 3H), 8.59(t, ³*J*_{HH}=6.0Hz, 3H), 7.71(t, ³*J*_{HH}=5.0Hz, 3H), 6.79(s, 1H), 4.03(s, 2H), 3.71-3.67(m, 12H), 3.55-3.52(m, 6H), 3.42-3.39(m, 6H), 2.43(t, ³*J*_{HH}=5.5Hz, 6H), 1.49(s, 27H), 1.48(s, 27H)

### <Synthesis of cell-membrane permeable molecule G3>

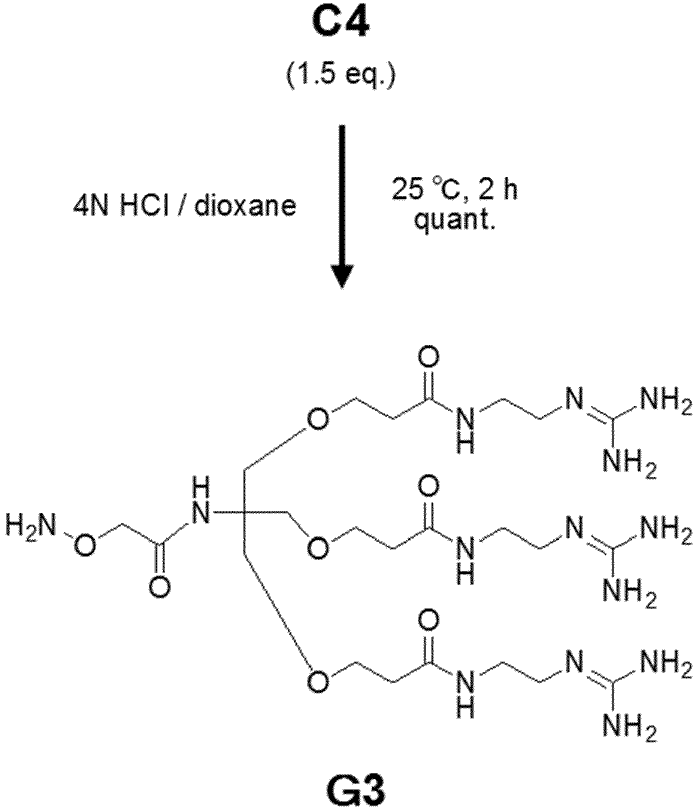

As presented in the above reaction scheme, cell-membrane permeable molecule G3 expressed by the above structural formula was synthesized.

Specifically, a dioxane hydrochloride solution (4N; 1.0 mL) was added to Compound C4 to prepare a solution, and the solution was stirred for 2 hours at 25°C. A white solid that precipitated was obtained through centrifugation, and further washed with diethyl ether (three times with 3 mL per time) to obtain cell-membrane permeable molecule G3 (90 mg, quantitative yield).

Identification data of the Compound G3 through Matrix Assisted Laser Desorption/Ionization Time-of-Flight Mass Spectrometry (MALDI-TOF MS) were as follows.

MALDI-TOF MS C₂₄H₅₀N₁₄O₈ calculated (M+H+) 663.401, found 663.402

### <Synthesis of Compound C5>

Solid-phase synthesis using microwaves was performed to synthesize a peptide having the following sequence on a Rink Amide resin (0.2 mmol/g).

Fmoc-Ahx-Cys-Met-Leu-Tyr-Ile-Val-Pro-Tyr-Phe-Ser-Val-Gly-Cys-NH₂

[In the above sequence, "Fmoc" denotes a 9-fluorenylmethyloxycarbonyl group and "Ahx" denotes a 6-aminohexanoic acid.]

The resin on which the peptide had been formed was immersed in trifluoroacetic acid (TFA)/water/triisopropylsilane/3,6-dioxa- 1,8-octanedithiol (92.5/2.5/2.5/2.5 (volume ratio)) for 3 hours to cleave the peptide from the resin.

The obtained peptide (55.0 mg, 30.0 µmol) was dissolved in DMF (2.5 mL). 1,3-Dibromo-2-propanone (20 mM DMF solution; 1.5 mL, 30 µmol) and N-methylmorpholine (10 mM DMF solution; 6.0 mL, 60 µmol) were added to the solution, and the mixture was stirred at 25°C for 1 hour. Diethyl ether (100 mL) was added thereto, followed by removal of the supernatant. The residue was washed with diethyl ether (100 mL) to obtain Compound C5 as a white solid (54.6 mg, 28.9 µmol, yield: 97%).

"CMLYIVPYFSVGC" in the structural formula of Compound C5 denotes an amino acid sequence of the peptide.

Identification data of the Compound C5 through Matrix Assisted Laser Desorption/Ionization Time-of-Flight Mass Spectrometry (MALDI-TOF MS) were as follows.

MALDI-TOF MS C₉₄H₁₂₈N₁₅O₂₀S₃⁺ calculated (M+H+) 1882.862, found 1883.140

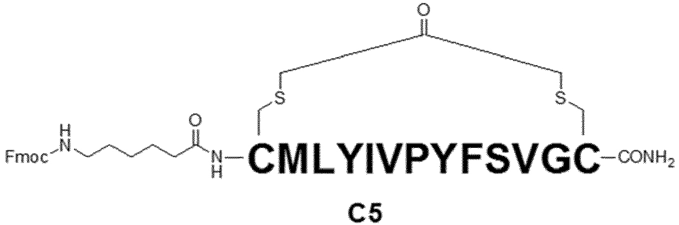

### <Synthesis of Compound C6>

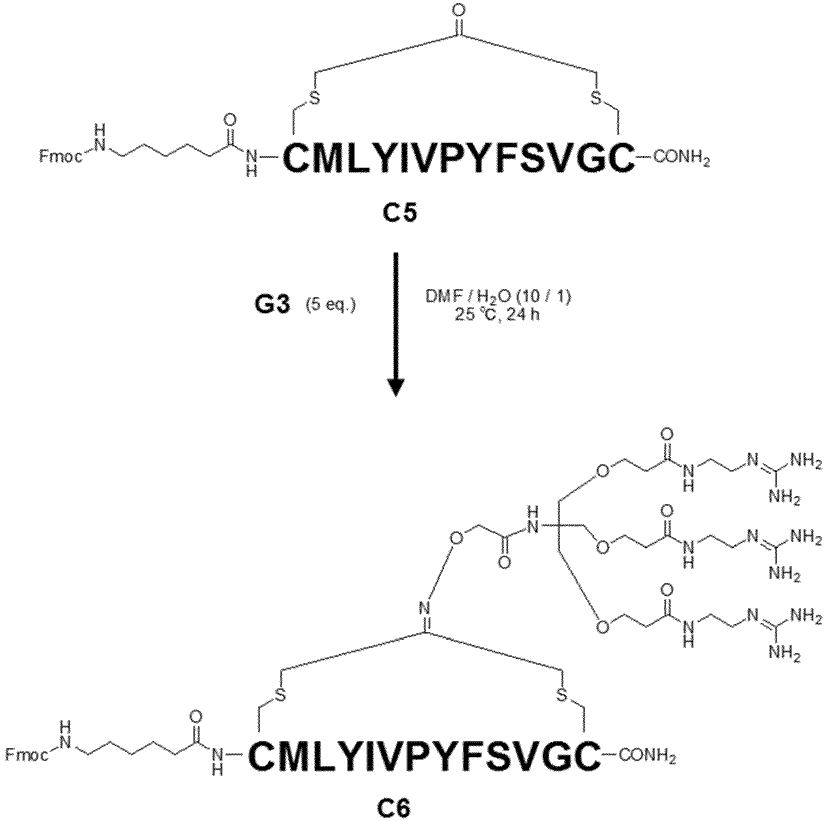

As presented in the above reaction scheme, Compound C6 expressed by the above structural formula was synthesized.

Specifically, Compound C5 (30 mg, 0.0159 mmol), cell-membrane permeable molecule G3 (52.7 mg, 0.0796 mmol), DMF (0.5 mL), and water (0.05 mL) were mixed, and the mixture was stirred at 25°C for 24 hours. Diethyl ether (5 mL) was added thereto, and the mixture was centrifuged, followed by removal of the supernatant. The residue was washed with diethyl ether (three times with 3 mL per time) to obtain a crude product of Compound C6. The crude product was purified through reversed-phase HPLC (high performance liquid chromatography) and freeze-dried to obtain Compound C6 (3.8 mg, 0.00150 mmol, yield: 9%).

### <Synthesis of peptide complex G3-P>

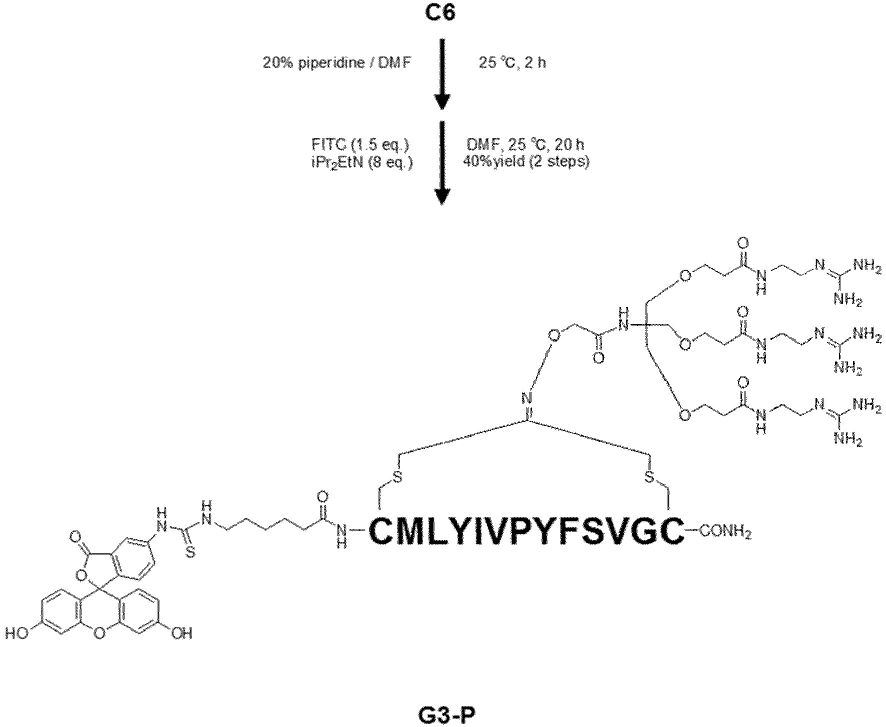

As presented in the above reaction scheme, peptide complex G3-P expressed by the above structural formula was synthesized.

Specifically, Compound C6 and a 20% piperidine/DMF solution (1.0 mL) were mixed, and the mixture was stirred at 25°C for 2 hours using a vortex mixer. Nitrogen gas was blown to volatilize the solvent. The residue was washed with diethyl ether (three times with 1 mL per time). With this, fluorescein isothiocyanate (FITC) (0.8 mg, 0.002 mmol), diisopropylethylamine (iPr₂EtN) (0.0022 mL, 0.0124 mmol), and DMF (0.5 mL) were mixed, and the mixture was stirred at 25°C for 20 hours. Diethyl ether (15 mL) was added thereto, and the mixture was centrifuged, followed by removal of the supernatant. The residue was washed with diethyl ether (twice with 5 mL per time) to obtain a crude product of peptide complex G3-P. The crude product was purified through reversed-phase HPLC and freeze-dried to obtain the peptide complex G3-P (1.6 mg, 0.00059 mmol, yield: 40%) as a white solid.

Identification data of the peptide complex G3-P through MALDI-TOF MS were as follows.

MALDI-TOF MS C₁₂₄H₁₇₇N₃₀O₃₀S₄⁺ calculated (M+H+) 2694.212, found 2694.208

### (Example 1: Synthesis of peptide complex G6-P)

### <Synthesis of Compound C7>

Solid-phase synthesis using microwaves was performed to synthesize a peptide having the following sequence on a Wang resin (0.66 mmol/g).

Fmoc-AOAA-Ahx-Glu

[In the above sequence, "AOAA" denotes aminooxyacetic acid and "Ahx" denotes 6-aminohexanoic acid.]

The resin on which the peptide had been formed was immersed in trifluoroacetic acid (TFA)/water/triisopropylsilane/3,6-dioxa- 1,8-octanedithiol (92.5/2.5/2.5/2.5 (volume ratio)) for 3 hours to cut out the peptide from the resin. Nitrogen gas was blown to remove the solvent. The residue was washed with diethyl ether (10 mL) to obtain Compound C7 expressed by the following structural formula as a white solid (quantitative yield).

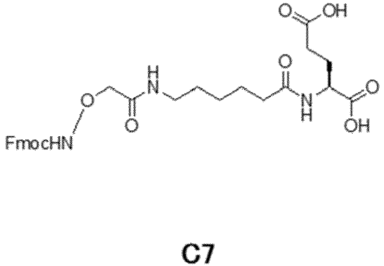

### <Synthesis of Compound C8>

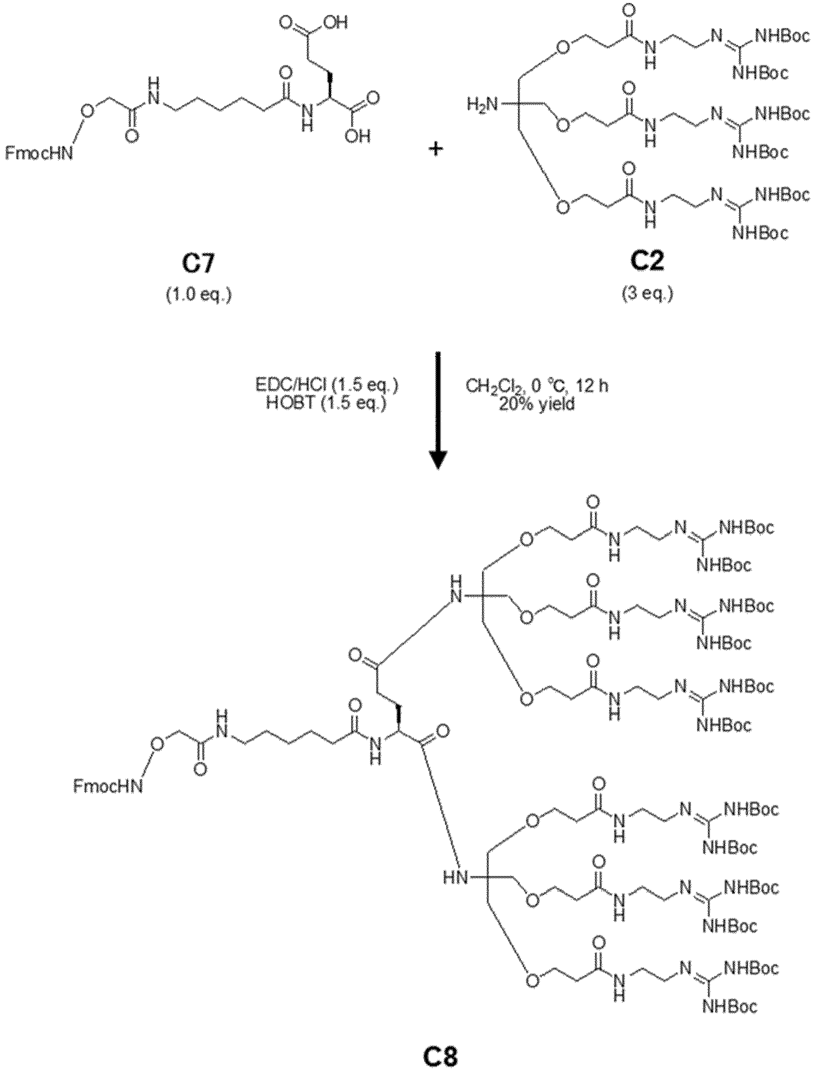

As presented in the above reaction scheme, Compound C8 expressed by the above structural formula was synthesized.

Specifically, Compound C7 (72.6 mg, 0.130 mmol) was dissolved in methylene chloride (3 mL), and the solution was cooled to 0°C. Compound C2 (466 mg, 0.392 mmol), EDC/HCl (75.1 mg, 0.392 mmol), and HOBT (52.9 mg, 0.392 mmol) were added thereto, and the mixture was stirred at 0°C for 12 hours. A saturated sodium chloride solution (20 mL) was added, and the mixture was extracted with methylene chloride (three times with 20 mL per time). The organic phase was dried with magnesium sulfate, followed by filtration and then concentration. The residue was purified through PTLC (methylene chloride/MeOH=10/1; Rf=0.7) to obtain Compound C8 (74.0 mg, 0.0255 mmol, yield: 20%) as colorless oily matter.

### <Synthesis of Compound C9>

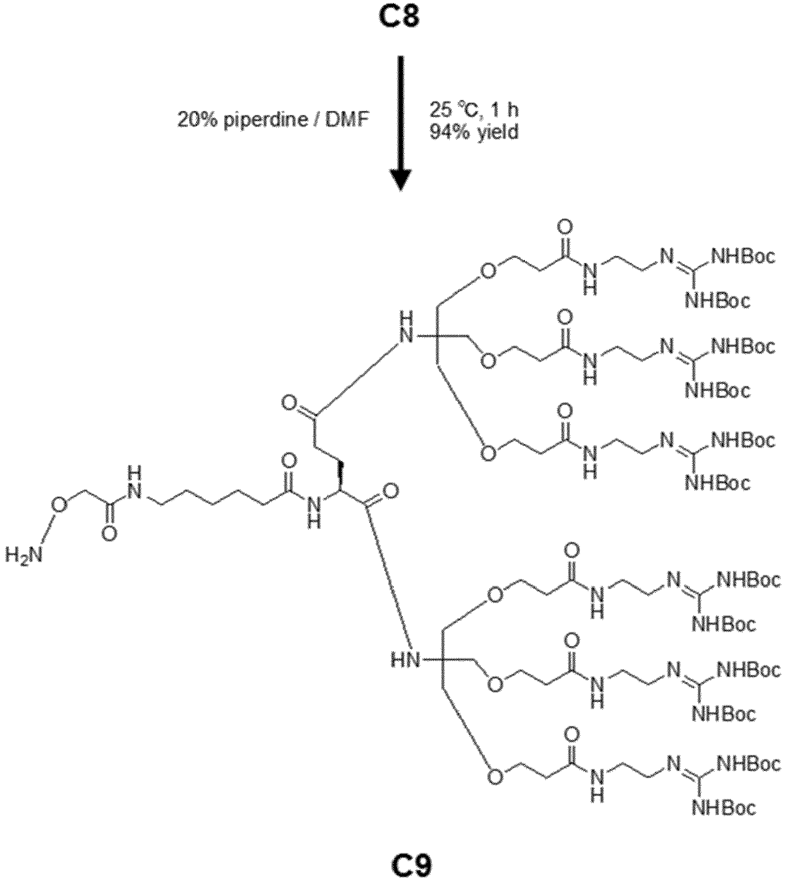

As presented in the above reaction scheme, Compound C9 expressed by the above structural formula was synthesized.

Specifically, Compound C8 (74 mg, 25.5 mmol) and a 20% piperidine/DMF solution (1.0 mL) were mixed, and the mixture was stirred at 25°C for 1 hour. Nitrogen gas was blown to the reaction liquid to volatilize the solvent to obtain a crude product of Compound C9 as colorless transparent oily matter.

### <Synthesis of cell-membrane permeable molecule G6>

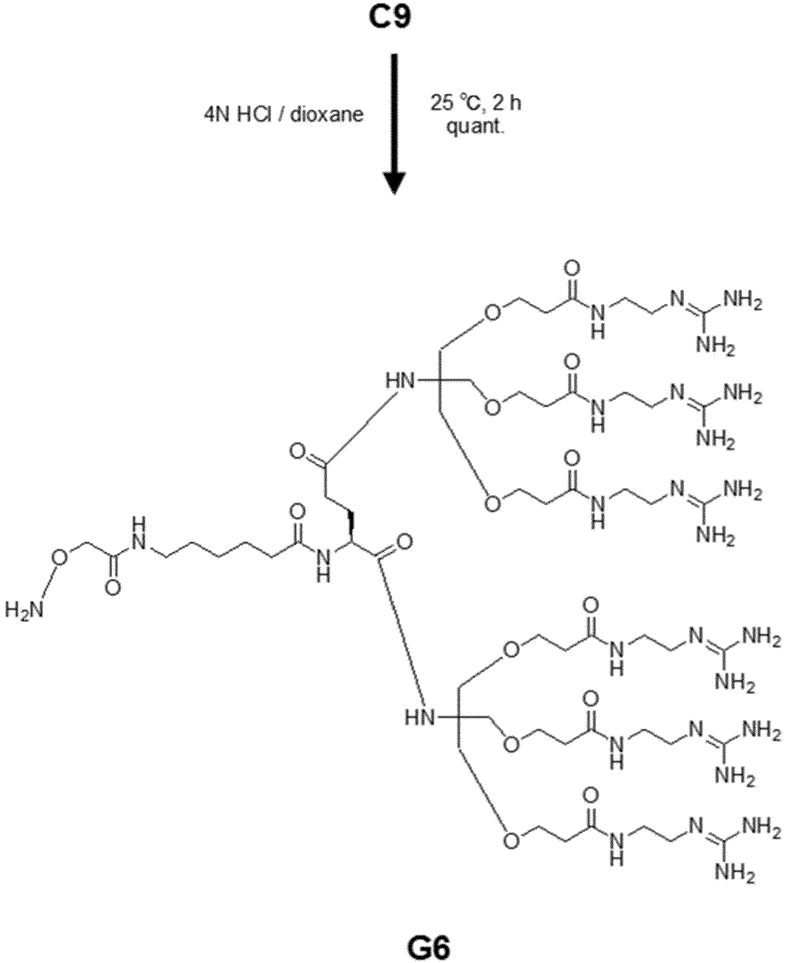

As presented in the above reaction scheme, cell-membrane permeable molecule G6 expressed by the above structural formula was synthesized.

Specifically, a dioxane hydrochloride solution (4N; 1.0 mL) was added to the crude product of Compound C9 to prepare a solution, and the solution was stirred for 2 hours at 25°C. A white solid that precipitated was obtained through centrifugation, and further washed with diethyl ether (three times with 1 mL per time) to obtain cell-membrane permeable molecule G6 (117 mg, quantitative yield) as a white solid.

### <Synthesis of Compound C10>

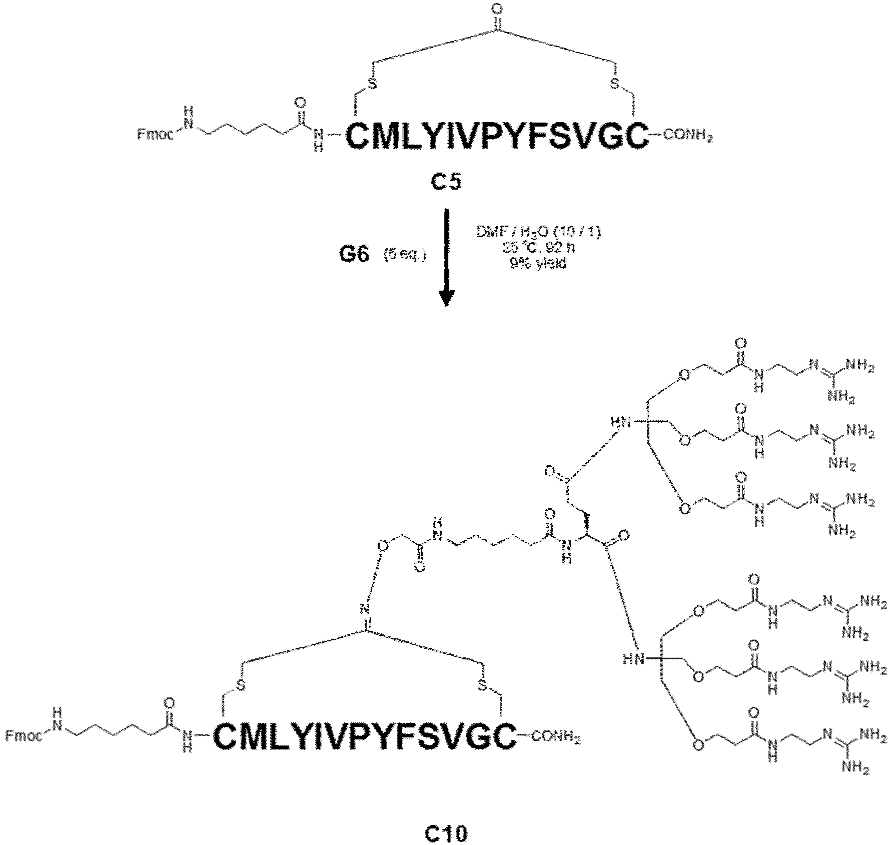

As presented in the above reaction scheme, Compound C10 expressed by the above structural formula was synthesized.

Specifically, Compound C5 (30 mg, 0.0159 mmol), cell-membrane permeable molecule G6 (117 mg, 0.0796 mmol), DMF (0.5 mL), and water (0.05 mL) were mixed, and the mixture was stirred at 25°C for 92 hours. Diethyl ether (5 mL) was added thereto, and the mixture was centrifuged, followed by removal of the supernatant. The residue was washed with diethyl ether (three times with 3 mL per time) to obtain a crude product of Compound C10. The crude product was purified through reversed-phase HPLC and freeze-dried to obtain Compound C10 (4.7 mg, 0.00141 mmol, yield: 9%) as a white solid.

### <Synthesis of peptide complex G6-P>

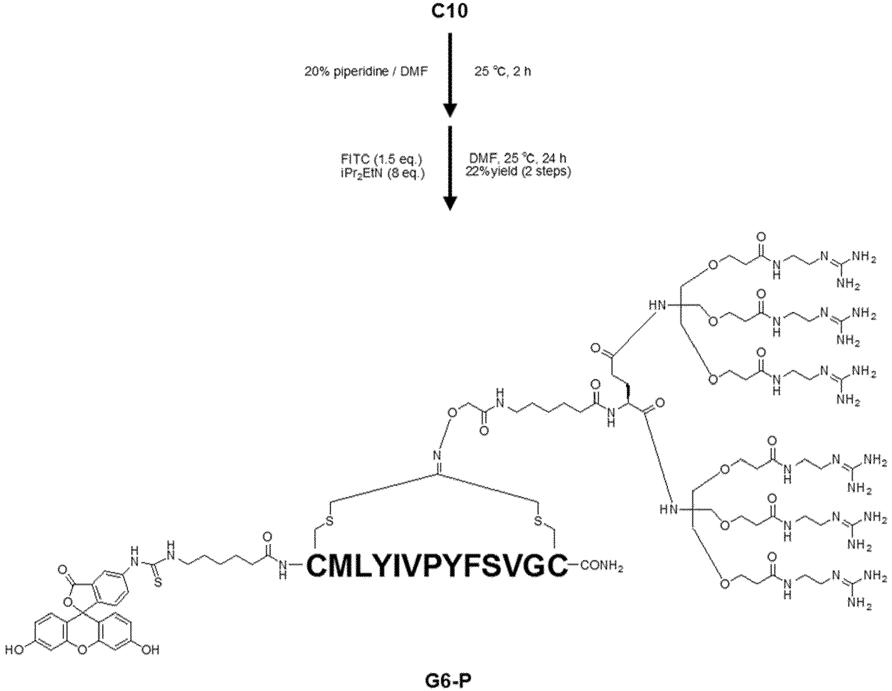

As presented in the above reaction scheme, peptide complex G6-P expressed by the above structural formula was synthesized.

Specifically, Compound C10 and a 20% piperidine/DMF solution (1.0 mL) were mixed, and the mixture was stirred at 25°C for 2 hours using a vortex mixer. Nitrogen gas was blown to volatilize the solvent. The residue was washed with diethyl ether (three times with 1 mL per time). With this, FITC (0.8 mg, 0.002 mmol), diisopropylethylamine (0.0022 mL, 0.0124 mmol), and DMF (0.5 mL) were mixed, and the mixture was stirred at 25°C for 24 hours. Diethyl ether (15 mL) was added thereto, and the mixture was centrifuged, followed by removal of the supernatant. The residue was washed with diethyl ether (twice with 5 mL per time) to obtain a crude product of peptide complex G6-P. The crude product was purified through reversed-phase HPLC and freeze-dried to obtain the peptide complex G6-P (1.1 mg, 0.00031 mmol, yield: 22%) as a yellow solid.

Identification data of the peptide complex G6-P through MALDI-TOF MS were as follows.

MALDI-TOF MS C₁₅₇H₂₄₀N₄₅O₃₉S₄⁺ calculated (M+H+) 3507.706, found 3507.696

### (Example 2: Synthesis of peptide complex G9-P)

### <Synthesis of Compound C11>

Solid-phase synthesis using microwaves was performed to synthesize a peptide having the following sequence on a Wang resin (0.66 mmol/g).

Fmoc-AOAA-Ahx-Glu-Glu

[In the above sequence, "AOAA" denotes aminooxyacetic acid and "Ahx" denotes 6-aminohexanoic acid.]

The resin on which the peptide had been formed was immersed in trifluoroacetic acid (TFA)/water/triisopropylsilane/3,6-dioxa- 1,8-octanedithiol (92.5/2.5/2.5/2.5 (volume ratio)) for 3 hours to cut out the peptide from the resin. Nitrogen gas was blown to remove the solvent. The residue was washed with diethyl ether (10 mL) to obtain Compound C11 expressed by the following structural formula as a white solid (quantitative yield).

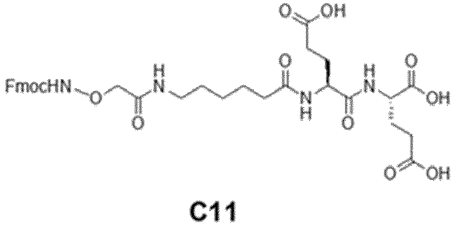

### <Synthesis of Compound C12>

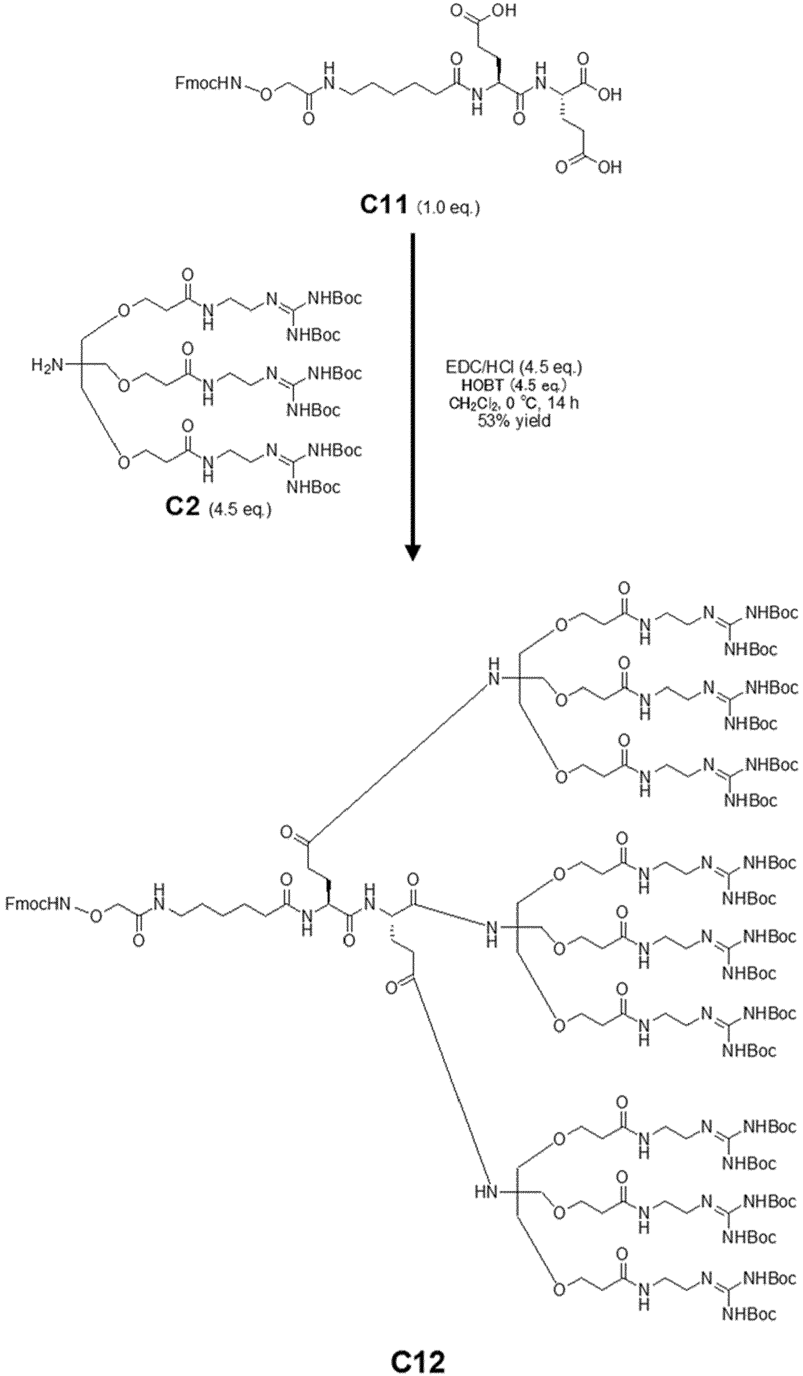

As presented in the above reaction scheme, Compound C12 expressed by the above structural formula was synthesized.

Specifically, methylene chloride (10 mL) was added to Compound C11 (50 mg, 0.073 mmol) to prepare a solution, and the solution was cooled to 0°C. To this, Compound C2 (391 mg, 0.329 mmol), EDC/HCl (63.0 mg, 0.329 mmol), and HOBT (44.1 mg, 0.329 mmol) were sequentially added, and the mixture was stirred at 0°C for 14 hours. Water (30 mL) was added to separate an organic layer. An aqueous layer was further extracted with methylene chloride (twice with 30 mL per time), and the organic layers were mixed. The organic layer was dried with magnesium sulfate, followed by filtration and concentration, to obtain a crude product of Compound C12. The crude product was purified through PTLC (methylene chloride/MeOH=10/1; Rf=0.5) to obtain Compound C12 (163 mg, 0.038 mmol, yield: 53%) as a white solid.

### <Synthesis of Compound C13>

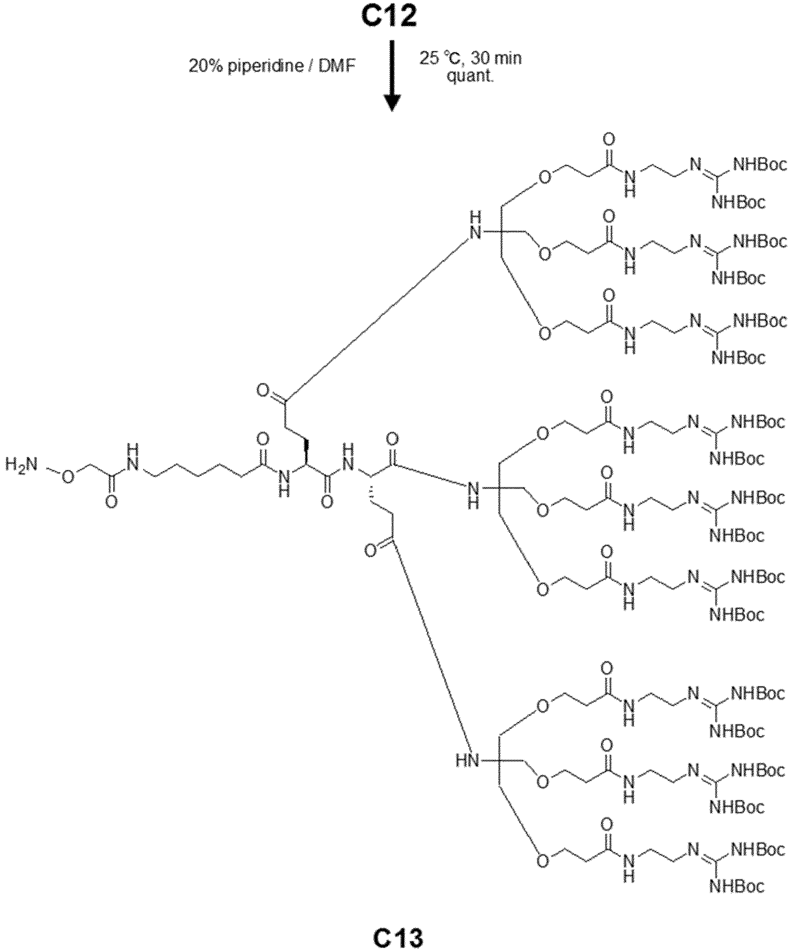

As presented in the above reaction scheme, Compound C13 expressed by the above structural formula was synthesized.

Specifically, Compound C12 (93 mg, 22.1 mmol) and a 20% piperidine/DMF solution (1.0 mL) were mixed to prepare a solution, and the solution was stirred at 25°C for 30 minutes. Nitrogen gas was blown to the reaction liquid to volatilize the solvent. The residue was washed with hexane (three times with 5 mL per time) to obtain Compound C13 (101 mg, quantitative yield) as a white solid.

### <Synthesis of cell-membrane permeable molecule G9>

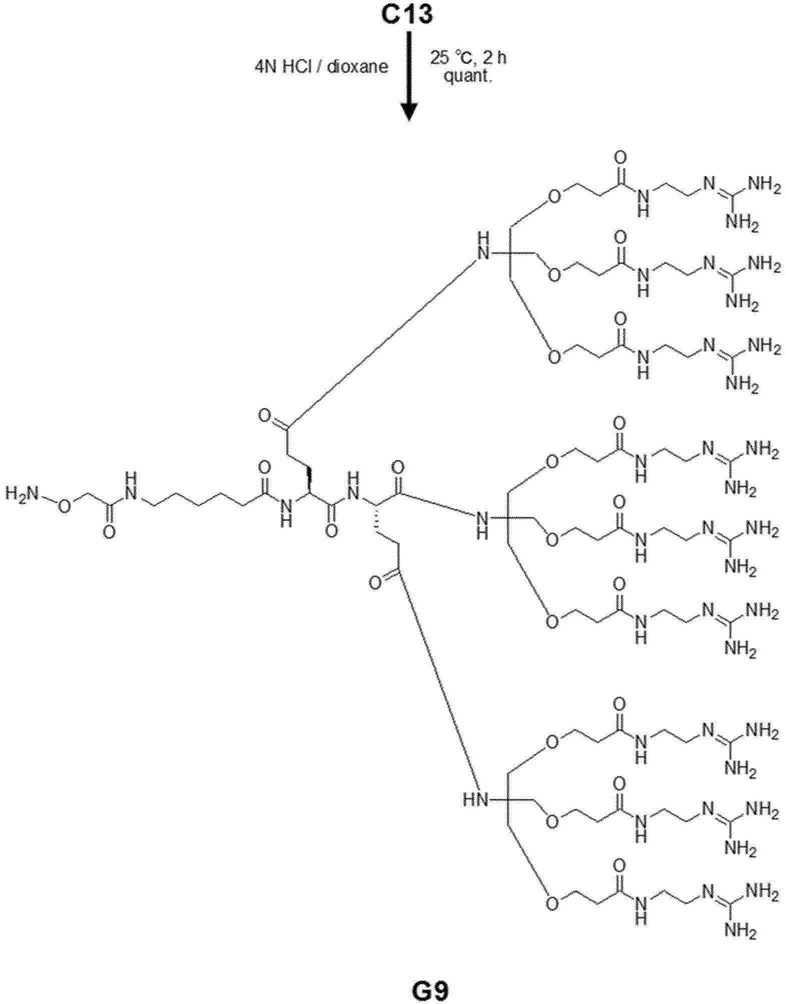

As presented in the above reaction scheme, cell-membrane permeable molecule G9 expressed by the above structural formula was synthesized.

Specifically, Compound C13 and a dioxane hydrochloride solution (4N; 1.0 mL) were mixed to prepare a solution, and the solution was stirred at 25°C for 2 hours. A white solid that precipitated was obtained through centrifugation, and further washed with diethyl ether (three times with 5 mL per time) to obtain cell-membrane permeable molecule G9 (148 mg, quantitative yield) as a white solid.

### <Synthesis of Compound C14>

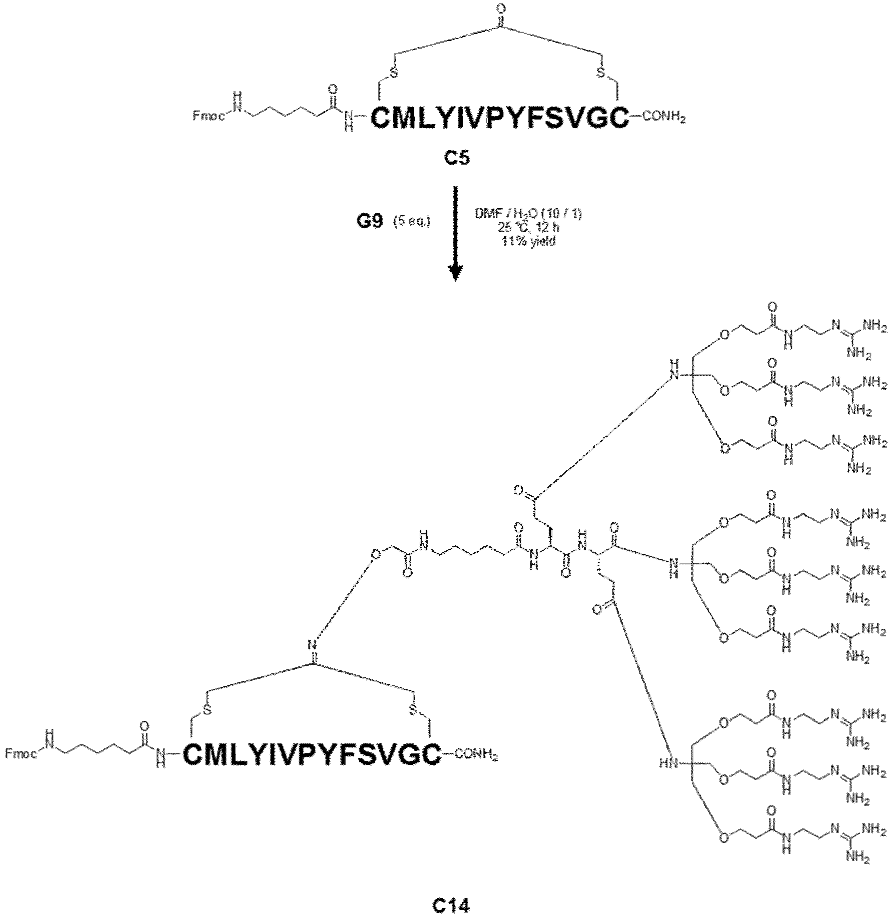

As presented in the above reaction scheme, Compound C14 expressed by the above structural formula was synthesized.

Specifically, Compound C5 (28 mg, 0.0148 mmol), cell-membrane permeable molecule G9 (161 mg, 0.074 mmol), DMF (0.5 mL), and water (0.05 mL) were mixed to prepare a solution. The solution was stirred at 25°C for 12 hours using a vortex mixer, and then nitrogen gas was blown to volatilize the solvent. The residue was washed with diethyl ether (three times with 5 mL per time) to obtain a crude product of Compound C14. The crude product was purified through reversed-phase HPLC and freeze-dried to obtain Compound C14 (6.6 mg, 0.00163 mmol, yield: 11%) as a white solid.

### <Synthesis of peptide complex G9-P>

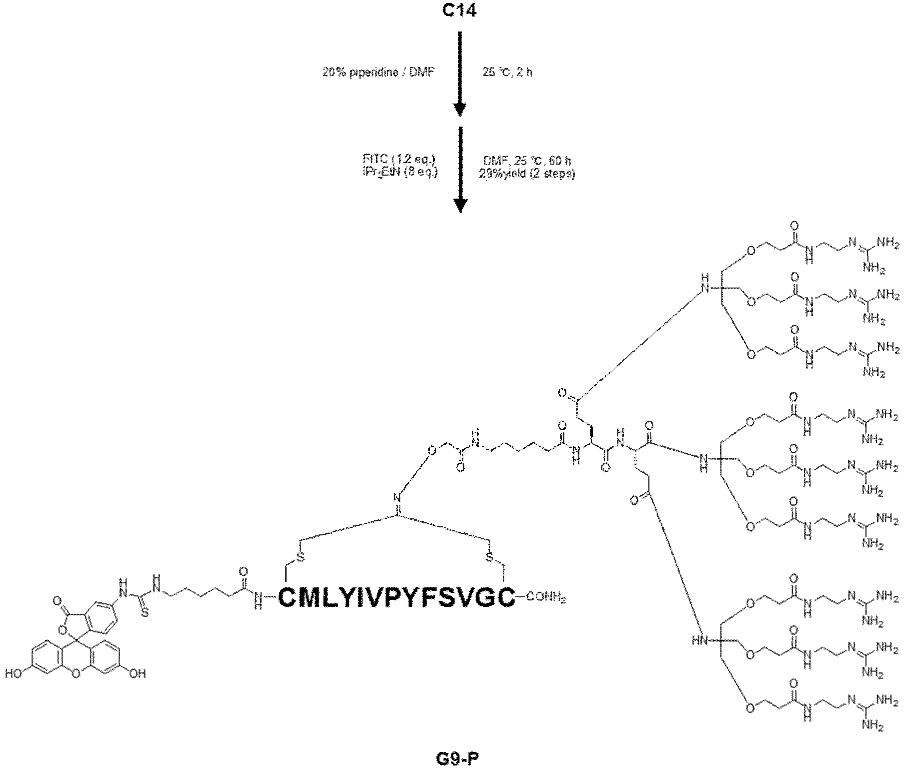

As presented in the above reaction scheme, peptide complex G9-P expressed by the above structural formula was synthesized.

Specifically, Compound C14 and a 20% piperidine/DMF solution (1 mL) were mixed, and the mixture was stirred at 25°C for 2 hours using a vortex mixer. Nitrogen gas was blown to volatilize the solvent. The residue was washed with diethyl ether (three times with 1 mL per time). With this, FITC (0.8 mg, 0.002 mmol), diisopropylethylamine (0.0022 mL, 0.0124 mmol), and DMF (0.5 mL) were mixed, and the mixture was stirred at 25°C for 60 hours. Diethyl ether (15 mL) was added thereto, and the mixture was centrifuged, followed by removal of the supernatant. The residue was washed with diethyl ether (twice with 5 mL per time) to obtain a crude product of peptide complex G9-P. The crude product was purified through reversed-phase HPLC and freeze-dried to obtain peptide complex G9-P (2.0 mg, 0.000475 mmol, yield: 29%) as a yellow solid.

Identification data of the peptide complex G9-P through MALDI-TOF MS were as follows.

MALDI-TOF MS C₁₈₄H₂₉₁N₅₉O₄₇S₄ calculated (M+H+) 4208.115, found 4208.107

### (Test Example 1: Evaluation of cell-membrane permeability)

HeLa cells (Human cervix adenocarcinoma cells) were cultured for 1 hour in a cell culture containing 2 µM of the peptide complex synthesized in Example 1 or 2 or Comparative Example 1 under conditions of 5% (v/v) CO₂ and 37°C. For culturing the HeLa cells, FluoroBrite D-MEM (obtained from ThermoFisher Co.) (supplemented with 10% (v/v) FCS (fetal calf serum) and 10% (v/v) GlutaMax (obtained from ThermoFisher Co.)) was used.

Next, the surfaces of the cells were washed with D-PBS (-) (supplemented with heparin (20 units/mL)), followed by recovery of the cells. The cells were suspended in D-PBS(-) (supplemented with 0.5% (v/v) BSA (bovine serum albumin), 200 mM EDTA (ethylenediaminetetraacetic acid), and 0.2% (v/v) propidium iodide (obtained from Sigma-Aldrich Co.)). A flow cytometer (BD FACS AriaIII) was used to measure fluorescence intensity. Upon flow cytometry analysis, the mode of fluorescence intensity was calculated for a population of living cells excluding dead cells that were propidium iodide-positive. As a control, dimethyl sulfoxide (DMSO) not containing the peptide complex was tested in the same manner. The results are presented in Table 1.

**Table 1**

| | Mode of fluorescence intensity | |
|---|---|---|
| | 0 hour of culture | 1 hour of culture |
| Comparative Example 1 | 66.6 | 412 |
| Example 1 | 60 | 730 |
| Example 2 | 65.8 | 877 |
| DMSO | 56.3 | 54 |

As presented in Table 1, at 1 hour of culture, the peptide complexes synthesized in Examples 1 and 2 both exhibited higher levels of cell-membrane permeation than the peptide complex synthesized in Comparative Example 1. This demonstrated that the peptide complex can enter cells in a short period of time by using a dendrimer-type cell-membrane-permeability-imparting group including a dendritic structure including four or more branches where at least four branches of the four or more branches include basic functional groups at ends thereof.

Aspects of the present invention are as follows, for example.
<1> A peptide complex including:
   a peptide; and
   a cell-membrane-permeability-imparting group introduced into the peptide,
   wherein the cell-membrane-permeability-imparting group includes a dendritic structure including four or more branches, and
   at least four branches of the four or more branches include basic functional groups at ends thereof.
<2> The peptide complex according to <1> above, wherein the basic functional groups include four or more guanidino groups.
<3> The peptide complex according to <1> or <2> above, wherein the peptide includes 200 or less amino acid residues.
<4> The peptide complex according to any one of <1> to <3> above, wherein the cell-membrane-permeability-imparting group includes a dendritic structural unit represented by General Formula (C) below: 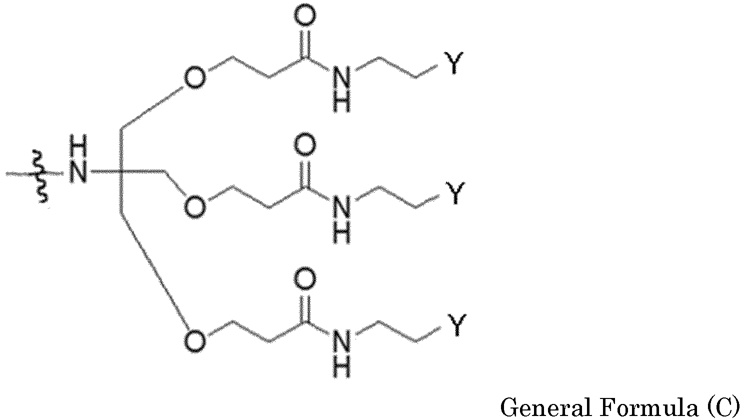 where in the General Formula (C), "Y" denotes the basic functional group.
<5> The peptide complex according to any one of <1> to <4> above, wherein the cell-membrane-permeability-imparting group is expressed by a structural formula below: 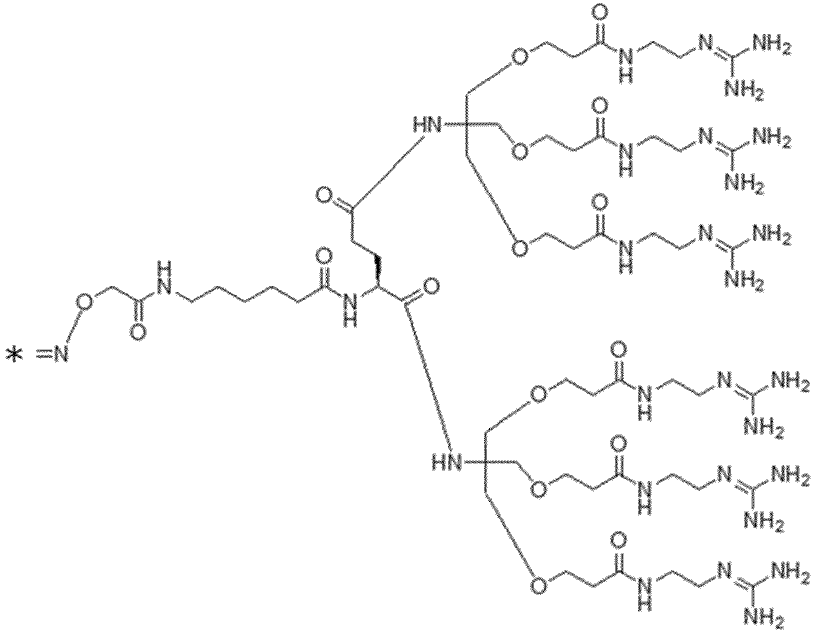 where in the structural formula, "*" denotes is a linking site to the peptide.
<6> The peptide complex according to any one of <1> to <4> above, wherein the cell-membrane-permeability-imparting group is expressed by a structural formula below: 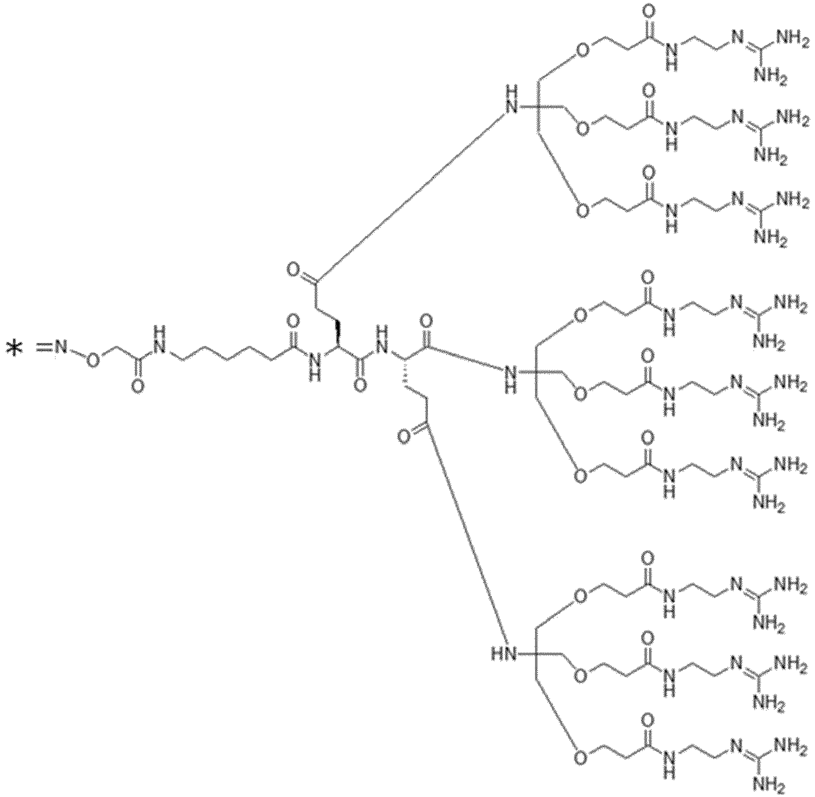 where in the structural formula, "*" denotes is a linking site to the peptide.
<7> A method for producing a peptide complex, the method including
   introducing a cell-membrane permeable molecule into a peptide,
   wherein the cell-membrane permeable molecule includes a dendritic structure including four or more branches, and at least four branches of the four or more branches include basic functional groups at ends thereof.
<8> The method for producing a peptide complex according to <7> above, wherein the basic functional groups include four or more guanidino groups.
<9> The method for producing a peptide complex according to <7> or <8> above, wherein the peptide includes 200 or less amino acid residues.
<10> The method for producing a peptide complex according to any one of <7> to <9> above, wherein the cell-membrane-permeability-imparting group includes a dendritic structural unit represented by General Formula (C) below: 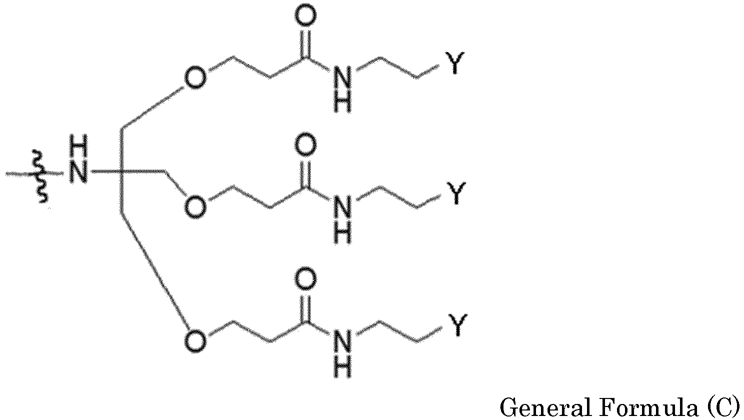 where in the General Formula (C), "Y" denotes the basic functional group.
<11> The method for producing a peptide complex according to any one of <7> to <10> above, wherein the cell-membrane-permeability-imparting group is expressed by a structural formula below: 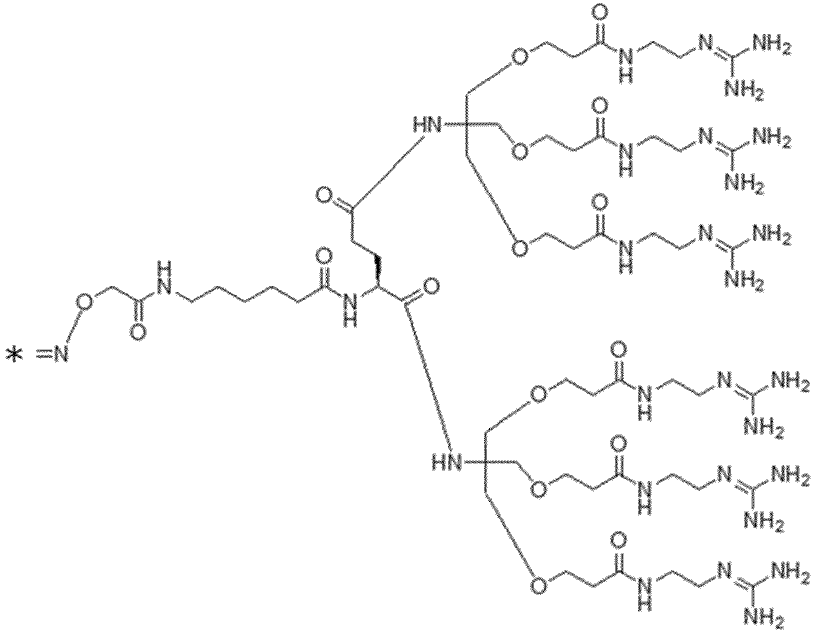 where in the structural formula, "*" denotes is a linking site to the peptide.
<12> The method for producing a peptide complex according to any one of <7> to <10> above, wherein the cell-membrane-permeability-imparting group is expressed by a structural formula below: 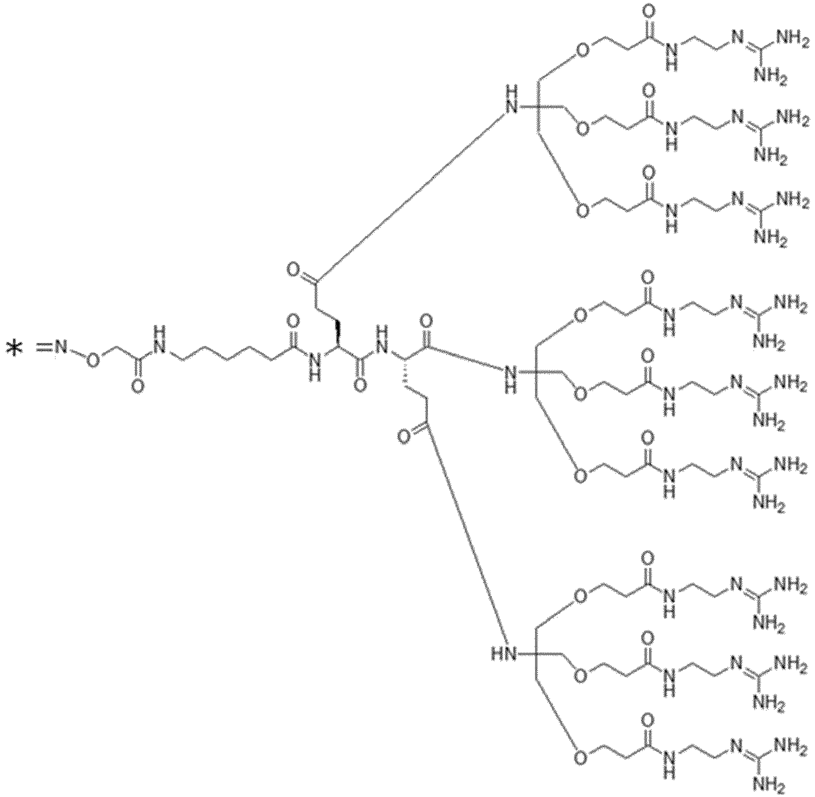 where in the structural formula, "*" denotes is a linking site to the peptide.
<13> A peptide library including
   the peptide complex defined in any one of claims <1> to <6>.
<14> A method for screening for a functional peptide, the method including
   screening for the functional peptide using the peptide library defined in <13> above.

## Claims

1. A peptide complex comprising:
a peptide; and
a cell-membrane-permeability-imparting group introduced into the peptide,
wherein the cell-membrane-permeability-imparting group comprises a dendritic structure comprising four or more branches, and
at least four branches of the four or more branches comprise basic functional groups at ends thereof.

2. The peptide complex according to claim 1, wherein the basic functional groups comprise four or more guanidino groups.

3. The peptide complex according to claim 1 or 2, wherein the peptide comprises 200 or less amino acid residues.

4. A method for producing a peptide complex, the method comprising
introducing a cell-membrane permeable molecule into a peptide,
wherein the cell-membrane permeable molecule comprises a dendritic structure comprising four or more branches, and at least four branches of the four or more branches comprise basic functional groups at ends thereof.

5. The method for producing a peptide complex according to claim 4, wherein the basic functional groups comprise four or more guanidino groups.

6. The method for producing a peptide complex according to claim 4 or 5, wherein the peptide comprises 200 or less amino acid residues.

7. A peptide library comprising
the peptide complex defined in any one of claims 1 to 3.

8. A method for screening for a functional peptide, the method comprising
screening for the functional peptide using the peptide library defined in claim 7.


SEQUENCE LISTING

<110>  KANEKA CORPORATION

<120>  PEPTIDE COMPLEX AND METHOD FOR PRODUCING THE SAME, AND USE
       THE PEPTIDE COMPLEX

<130>  N-KK006-19P

<150>  JP 2019-064539
<151>  2019-03-28

<160>  1

<170>  PatentIn version 3.1

<210>  1
<211>  13
<212>  PRT
<213>  Artificial Sequence

<220>
<223>  Designed peptide

<400>  1
Cys Met Leu Tyr Ile Val Pro Tyr Phe Ser Val Gly Cys
1               5                   10